(19) Europäisches Patentamt
European Patent Office
Office européen des brevets

(11) **EP 3 971 280 A1**

(12) **EUROPEAN PATENT APPLICATION**
published in accordance with Art. 153(4) EPC

(43) Date of publication:
**23.03.2022 Bulletin 2022/12**

(21) Application number: **20832170.3**

(22) Date of filing: **15.06.2020**

(51) International Patent Classification (IPC):
**C12M 1/00** $^{(2006.01)}$   **C12M 3/00** $^{(2006.01)}$
**C12N 5/071** $^{(2010.01)}$   **C12N 5/10** $^{(2006.01)}$

(52) Cooperative Patent Classification (CPC):
**C12M 1/00; C12M 3/00;** C12N 5/10

(86) International application number:
**PCT/JP2020/023362**

(87) International publication number:
**WO 2020/262062 (30.12.2020 Gazette 2020/53)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.06.2019 JP 2019120416**

(71) Applicant: **FUJIFILM Corporation
Tokyo 106-8620 (JP)**

(72) Inventors:
• **OKU, Keisuke
  Ashigarakami-gun, Kanagawa 258-8577 (JP)**
• **ITO, Koju
  Ashigarakami-gun, Kanagawa 258-8577 (JP)**
• **NAKAZAWA, Koji
  Kitakyushu-shi, Fukuoka 808-0135 (JP)**

(74) Representative: **Hoffmann Eitle
Patent- und Rechtsanwälte PartmbB
Arabellastraße 30
81925 München (DE)**

(54) **METHOD FOR PRODUCING HEPATOCYTE CULTURE, CELL-CONTAINING POROUS FILM, AND METHOD FOR IMPROVING OR MAINTAINING FUNCTION OF HEPATOCYTE OR HEPATIC PROGENITOR CELL**

(57)    Provided are a method for producing a hepatocyte culture and a method for improving or maintaining a function of a hepatocyte or a hepatic progenitor cell, each method including a culturing step of culturing a hepatocyte or a hepatic progenitor cell inside at least a part of a plurality of opening pores and communication pores of a porous film having the plurality of opening pores provided on a surface thereof and the communication pores for communicating the opening pores adjacent to each other; and a cell-containing porous film.

FIG. 1A

**Description**

BACKGROUND OF THE INVENTION

1. Field of the Invention

The present disclosure relates to a method for producing a hepatocyte culture, a cell-containing porous film, and a method for improving or maintaining a function of a hepatocyte or a hepatic progenitor cell.

2. Description of the Related Art

Cultured hepatocytes are widely used in fields such as drug development and biological function research. Conventionally, hepatocytes have generally been cultured on the surface of a culture dish (also referred to as two-dimensional culture), but it has been difficult to sufficiently maintain functions such as metabolic characteristics of hepatocytes in two-dimensional culture. On the other hand, there has been known a method in which hepatocytes are cultured in a spheroid (aggregate) state to maintain cell characteristics (also referred to as three-dimensional culture). According to three-dimensional culture, it has been known that the functional expression of a living organism is improved by a cell-to-cell interaction.

With regard to the method for maintaining the function of hepatocytes, JP2007-319006A has proposed a method in which hepatocytes are retained and cultured on one or both sides of a honeycomb-like porous membrane made of a water-insoluble polymer and having through-holes.

In addition, JP2016-63948A has proposed a porous film for tissue culture intended to be indwelled in vivo.

In addition, WO2018/061846A has proposed a method for producing a cell tissue having a micro-order network structure using a porous membrane.

SUMMARY OF THE INVENTION

In the culture of hepatocytes, the functional expression of a living organism is improved in the three-dimensional culture as compared with the two-dimensional culture, but there is a problem such as sufficient nutrition being not distributed to the cells inside the spheroid. In addition, there is room for improvement in maintaining the function of hepatocytes even in the method using the porous membrane described in JP2007-319006A.

In view of the above circumstances, an object of the present disclosure is to provide a method for producing a hepatocyte culture capable of satisfactorily maintaining or improving functional characteristics of a hepatocyte, a cell-containing porous film, and a method for improving or maintaining a function of a hepatocyte or a hepatic progenitor cell.

The means for achieving the above-mentioned object include the following aspects.

[1] A method for producing a hepatocyte culture, the method comprising a culturing step of culturing a hepatocyte or a hepatic progenitor cell inside at least a part of a plurality of opening pores and communication pores of a porous film having the plurality of opening pores provided on a surface of the porous film and the communication pores for communicating the opening pores adjacent to each other.

[2] The production method according to [1], in which an average opening diameter of the plurality of opening pores is 10 μm or more and 500 μm or less.

[3] The production method according to [1], in which an average opening diameter of the plurality of opening pores is 20 μm or more and 200 μm or less.

[4] The production method according to any one of [1] to [3], in which the plurality of opening pores are arranged in a honeycomb shape on the surface of the porous film.

[5] The production method according to any one of [1] to [4], in which a coefficient of variation of opening diameters of the plurality of opening pores is 20% or less, and an opening ratio of the plurality of opening pores is 30% or more and 80% or less.

[6] The production method according to any one of [1] to [5], in which the porous film is a monolayer structure including one porous layer having the plurality of opening pores and the communication pores.

[7] The production method according to any one of [1] to [5], in which the porous film is a laminate including two or more porous layers having the plurality of opening pores and the communication pores, and average opening diameters of the plurality of opening pores are the same or different between the porous layers.

[8] The production method according to any one of [1] to [6], in which the porous film has a non-penetrating structure.

[9] The production method according to any one of [1] to [8], in which the hepatocyte or the hepatic progenitor cell is an iPS cell-derived differentiated cell, a human primary cell, or a non-human animal-derived primary cell.

[10] A cell-containing porous film comprising:

a porous film having a plurality of opening pores provided on a surface of the porous film and communication pores for communicating the opening pores adjacent to each other; and

hepatocytes or hepatic progenitor cells present inside at least a part of the plurality of opening pores and the communication pores of the porous film.

[11] The cell-containing porous film according to [10], in which the hepatocytes or the hepatic progenitor cells are present inside two or more adjacent opening pores among the plurality of opening pores.

[12] A method for improving or maintaining a function of a hepatocyte or a hepatic progenitor cell, the method comprising a culturing step of culturing a hepatocyte or a hepatic progenitor cell inside at least a part of a plurality of opening pores and communication pores of a porous film having the plurality of opening pores provided on a surface of the porous film and the communication pores for communicating the opening pores adjacent to each other.

[13] The method for improving or maintaining a function of a hepatocyte or a hepatic progenitor cell according to [12], in which an average opening diameter of the plurality of opening pores is 10 $\mu$m or more and 500 $\mu$m or less.

[14] The method for improving or maintaining a function of a hepatocyte or a hepatic progenitor cell according to [12], in which an average opening diameter of the plurality of opening pores is 20 $\mu$m or more and 200 $\mu$m or less.

[15] The method for improving or maintaining a function of a hepatocyte or a hepatic progenitor cell according to any one of [12] to [14], in which the plurality of opening pores are arranged in a honeycomb shape on the surface of the porous film.

[16] The method for improving or maintaining a function of a hepatocyte or a hepatic progenitor cell according to any one of [12] to [15], in which a coefficient of variation of opening diameters of the plurality of opening pores is 20% or less, and an opening ratio of the plurality of opening pores is 30% or more and 80% or less.

[17] The method for improving or maintaining a function of a hepatocyte or a hepatic progenitor cell according to any one of [12] to [16], in which the porous film is a monolayer structure including one porous layer having the plurality of opening pores and the communication pores.

[18] The method for improving or maintaining a function of a hepatocyte or a hepatic progenitor cell according to any one of [12] to [16], in which the porous film is a laminate including two or more porous layers having the plurality of opening pores and the communication pores, and average opening diameters of the plurality of opening pores are the same or different between the porous layers.

[19] The method for improving or maintaining a function of a hepatocyte or a hepatic progenitor cell according to any one of [12] to [17], in which the porous film has a non-penetrating structure.

[20] The method for improving or maintaining a function of a hepatocyte or a hepatic progenitor cell according to any one of [12] to [19], in which the hepatocyte or the hepatic progenitor cell is an iPS cell-derived differentiated cell, a human primary cell, or a non-human animal-derived primary cell.

According to the present disclosure, a method for producing a hepatocyte culture capable of satisfactorily maintaining or improving functional characteristics of a hepatocyte, a cell-containing porous film, and a method for improving or maintaining a function of a hepatocyte or a hepatic progenitor cell are provided.

BRIEF DESCRIPTION OF THE DRAWINGS

Fig. 1A is a perspective view showing a structure of a porous film in one embodiment.
Fig. 1B is a plan view seen from the opening surface side of the porous film in Fig. 1A.
Fig. 1C is a cross-sectional view of the porous film taken along a line c-c in Fig. 1B.
Fig. 1D is a cross-sectional view of the porous film taken along a line d-d in Fig. 1B.
Fig. 2 is a schematic diagram showing an example of a method for producing a porous film.
Fig. 3 shows a laser micrograph of a porous film in one embodiment.
Fig. 4A shows an example of a porous film used in a culture device.
Fig. 4B shows an example of a PMMA ring used in the culture device.
Fig. 4C shows an example of the culture device.
Fig. 5 shows a phase contrast microscope image during preculture of an iCell in Examples.
Fig. 6 shows a phase contrast image showing a cellular morphological change during main culture of an iCell in Examples.
Fig. 7 shows a fluorescence microscope image of staining of an actin skeleton of an iCell in Examples.
Fig. 8 shows an evaluation result of a function of an iCell in Examples.
Fig. 9 shows an evaluation result of a function of a primary rat hepatocyte in

Examples.

DESCRIPTION OF THE PREFERRED EMBODIMENTS

Hereinafter, embodiments of the present disclosure will be described. These descriptions and examples are only illustrative of the embodiments and do not limit the scope of the invention.

The numerical range indicated by using "to" in the present disclosure indicates a range including numerical values described before and after "to" as a lower limit value and an upper limit value, respectively.

The term "step" in the present disclosure not only includes an independent step, but also includes a step that may not be clearly distinguished from the other step but still achieves a desired effect of the step.

In the present disclosure, upon referring to an amount of each ingredient in a composition, the amount means a total amount of a plurality of substances present in the composition unless otherwise specified, in a case where a plurality of substances corresponding to each ingredient are present in the composition.

In the present disclosure, a combination of two or more preferred aspects is a more preferred aspect.

In the present disclosure, the coefficient of variation is expressed in terms of percentage. The coefficient of variation is a value obtained by dividing a standard deviation by a mean value for a certain population and is an indicator showing the degree of variability of the population.

In a case where an embodiment is described in the present disclosure with reference to the accompanying drawings, the configuration of the corresponding embodiment is not limited to the configuration shown in the drawings. In addition, the size of the member in each drawing is conceptual, and the relative relationship between the sizes of the members is not limited thereto. In addition, members having substantially the same function in each drawing are given the same reference numerals in all drawings, and any redundant description may be omitted.

<Porous film>

First, a porous film used in the method for producing a hepatocyte culture according to the present disclosure will be described in detail. The porous film has a plurality of opening pores provided on the surface thereof and communication pores for communicating the opening pores adjacent to each other. In the present disclosure, the term "opening pore" refers to a pore having an opening on at least one main surface of the porous film.

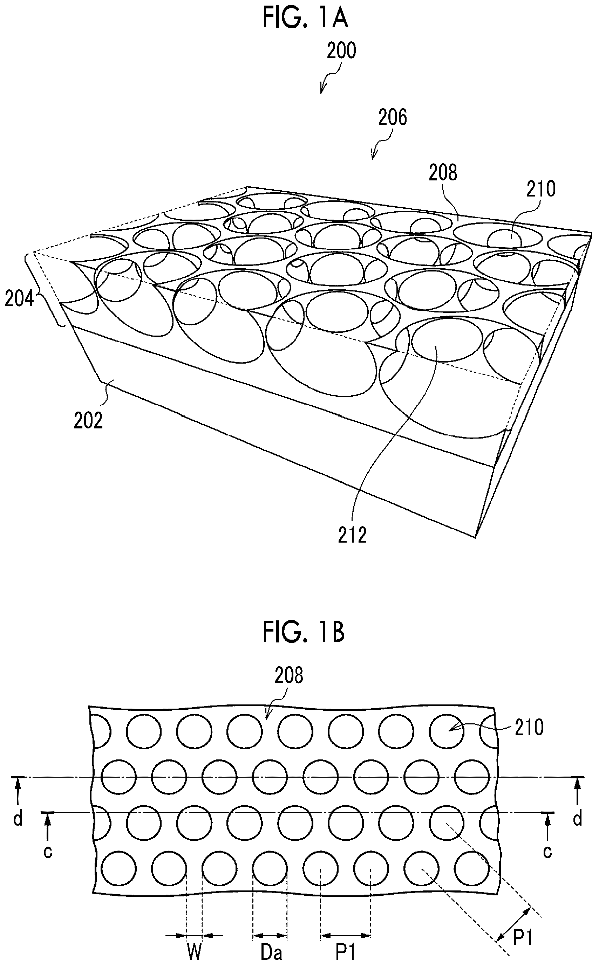

Hereinafter, an example of the porous film will be described with reference to Fig. 1A to Fig. 1D. The same reference numerals are given to the same or equivalent constituent elements and parts in each drawing.

In the following description, the term "plane direction" means a main plane direction of the porous film, and the term "thickness direction" means a thickness direction of the porous film.

In the following description, the "major axis" means a maximum length among any two-point distances on the contour, but in a case where the direction is specified, it means a maximum length among any two-point distances in that direction.

In the following description, the term "center of the opening" refers to a centroid in a case where the opening is taken as a two-dimensional graphic shape in a plane direction.

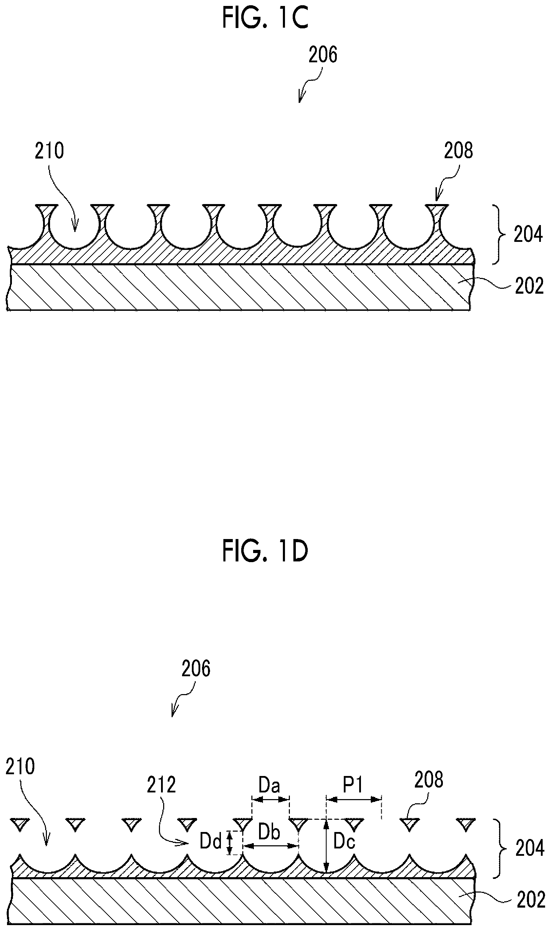

Fig. 1A is a perspective view showing the structure of the porous film, Fig. 1B is a plan view seen from the opening surface side of the porous film in Fig. 1A, Fig. 1C is a cross-sectional view of the porous film taken along a line c-c in Fig. 1B, and Fig. 1D is a cross-sectional view of the porous film taken along a line d-d in Fig. 1B.

Fig. 1A shows a porous film 200 which is an example of the porous film used in the method for producing a hepatocyte culture according to the present disclosure. The porous film 200 is a laminate including a porous layer 204 in which a plurality of opening pores 210 are arranged in a plane direction and a support 202 for supporting the porous layer 204. As another example, the porous film 200 may be a monolayer structure including one porous layer 204 without the support 202. As yet another example, the porous film 200 may be a laminate including two or more porous layers 204. In a case where the porous film 200 is a laminate including two or more porous layers 204, average opening diameters of a plurality of opening pores 210 provided on the surface of each porous layer 204 may be the same or different between the porous layers.

In the porous layer 204 of the porous film 200, the opening pores 210 are arranged over an entire area in a plane direction. However, in a case where the porous layer 204 has a region with which cells cannot come into contact, the opening pores 210 may not be arranged in the region with which the cells cannot come into contact.

An opening surface 206 of the porous film 200 is the surface on the side where the plurality of opening pores 210 are opened and is the surface on the side where the cells are seeded. The opening surface 206 refers to a surface which has a flat portion 208 and an opening and is coplanar with the flat portion. In the opening surface 206, the openings of the adjacent opening pores 210 are spaced from each other. In the present disclosure, an opening pore having one spaced opening on the opening surface 206 is referred to as one opening pore. A liquid culture medium is supplied to the inside of opening pores 210 and communication pores 212 through the openings of the opening pores 210 on the opening surface 206.

In Fig. 1A to Fig. 1D, the porous film 200 has a non-penetrating structure. That is, the plurality of opening pores 210 are bottomed pores that do not penetrate through the porous film 200. Since the porous film has a non-penetrating structure, the seeded cells can be suitably retained in the opening pores. The porous film 200 may have a non-penetrating structure by using the porous layer 204 through which the plurality of opening pores 210 do not penetrate, or may have a non-penetrating structure by laminating the support 202 even in a case the plurality of opening pores 210 penetrate through the porous layer 204. The porous film 200 shown in Fig. 1A to Fig. 1D has a non-penetrating structure, but the porous film 200 may have a penetrating structure as long as cells can be retained.

Examples of the shape of the opening pore 210 include a truncated spherical shape obtained by cutting out a part of a sphere, a barrel shape, a cylindrical shape, and a prismatic shape. Examples of the shape of the opening of the opening pore 210 include a circular shape, an elliptical shape, and a polygonal shape.

In Figs. 1A to 1D, the plurality of opening pores 210 are regularly arranged on the surface of the porous film, and specifically, are arranged in a honeycomb shape on the surface of the porous film. As another example, the plurality of opening pores 210 may be arranged in a lattice shape or a face-centered lattice shape. The arrangement of the plurality of opening pores 210 may be a random arrangement. From the viewpoint of increasing the uniformity of the density of the opening pores 210 in a plane direction and making it easy to obtain a homogeneous hepatocyte culture, it is preferable that the plurality of opening pores 210 are regularly arranged.

The honeycomb-like arrangement refers to an arrangement in which a parallel hexagonal shape (preferably a regular hexagonal shape) or a shape close thereto is used as a unit and the centroid of the opening is located at the apex and the point of intersection of diagonal lines of such a graphic shape. The lattice-like arrangement refers to an arrangement in which a parallelogram shape (including, needless to say, a square shape, a rectangle shape, a rhombus shape, preferably a square shape) or a shape close thereto is used as a unit, and the centroid of the opening is located at the apex of such a graphic shape. The face-centered lattice-like arrangement refers to an arrangement in which a parallelogram shape (including, needless to say, a square shape, a rectangle shape, a rhombus shape, preferably a square shape) or a shape close thereto is used as a unit, and the centroid of the opening is located at the apex and the point of intersection of diagonal lines of such a graphic shape.

As a reference guideline of being regularly arranged, there is an arrangement in which, regarding the area of the parallel hexagonal shape or the parallelogram shape which is the unit of arrangement, the coefficient of variation thereof is 10% or less. The coefficient of variation is obtained for any 10 units of arrangement.

In the porous film 200, the opening pores 210 adjacent to each other communicate with each other through the communication pores 212 inside the porous layer 204. One opening pore 210 preferably communicates with 50% by number or more of the adjacent opening pores, more preferably with 67% by number or more of the adjacent opening pores, still more preferably with 84% by number or more of the adjacent opening pores, and particularly preferably with all the adjacent opening pores 210. For example, in the porous film 200 in which a plurality of opening pores 210 are arranged in a honeycomb shape, one opening pore 210 preferably communicates with three or more opening pores 210, more preferably four or more opening pores 210, still more preferably five or more opening pores 210, and particularly preferably six opening pores 210, among the six adjacent opening pores 210. That is, one opening pore 210 preferably has three or more communication pores 212, more preferably four or more communication pores 212, still more preferably five or more communication pores 212, and particularly preferably six communication pores 212. Regarding the number of opening pores 210 communicating with each other or the number of communication pores, the average of 10 or more randomly selected opening pores 210 is preferably in the above range.

In the porous film 200, the communication pore 212 is provided as a pore formed at a portion where the wall surfaces of adjacent opening pores intersect, but as another example, the communication pore 212 may be provided as a tubular void space connecting the adjacent opening pores.

Hereinafter, the structural dimensions of of the porous film 200 will be described.

The plurality of opening pores 210 have a size that allows hepatocytes or hepatic progenitor cells seeded on the porous film 200 to enter and engraft. The average major axis of hepatocytes or hepatic progenitor cells seeded on the porous film 200 is, for example, 5 $\mu$m to 30 $\mu$m and preferably 10 $\mu$m to 20 $\mu$m. The average major axis of the cells is an average value of the major axes of any 10 or more cells in an observation visual field of a phase contrast microscope.

The opening diameter Da of the opening pore 210 is not particularly limited as long as the seeded hepatocytes or hepatic progenitor cells can enter the inside of the opening pore 210. For example, the average opening diameter is preferably 100% or more, more preferably 200% or more, and still more preferably 300% or more with respect to the average major axis of hepatocytes or hepatic progenitor cells seeded on the porous film 200. In addition, the average opening diameter is preferably 1000% or less, more preferably 900% or less, and still more preferably 800% or less with respect to the average major axis of hepatocytes or hepatic progenitor cells seeded on the porous film 200. In a case where the average opening diameter is 100% or more, more preferably 200% or more, and still more preferably 300% or more with respect to the average major axis of hepatocytes or hepatic progenitor cells seeded on the porous film 200, the hepatocytes or hepatic progenitor cells seeded on the porous film 200 can suitably enter the inside of the opening pore 210. In a case where the average opening diameter is 1000% or less, more preferably 900% or less, and still more preferably 800% or less with respect to the average major axis of hepatocytes or hepatic progenitor cells seeded on the porous film 200, it tends to be possible to spread a culture medium suitably to the cells. The opening diameter Da is defined as a major axis of the opening of the opening pore 210. In addition, the average opening diameter is defined as an average value of the opening diameter Da of 10 or more opening pores 210 randomly selected in the observation visual field with a laser microscope.

Specifically, the average opening diameter of the opening pore 210 is preferably 10 $\mu$m to 500 $\mu$m, more preferably 20 $\mu$m to 200 $\mu$m, and still more preferably 30 $\mu$m to 100 $\mu$m.

From the viewpoint of easily obtaining a homogeneous hepatocyte culture, the coefficient of variation of the opening diameter Da of the opening pore 210 is preferably 20% or less, and the smaller the coefficient of variation is, the more preferable. The coefficient of variation is obtained for the opening diameter Da of any 10 opening pores 210.

In the plan view of the opening surface 206 of the porous film 200, the ratio of a total area of the openings to a total area of a cell culture region (that is, a total area of the flat portions 208 and the openings) is preferably 30% to 80%, more preferably 35% to 75%, and still more preferably 40% to 70%.

Hereinafter, the ratio of a total area of the openings to a total area of the cell culture region in the plan view of the opening surface is also referred to as an "opening ratio". The cell culture region means a region with which cells can come into contact by seeding. Therefore, a region on the opening surface 206 of the porous film 200 with which cells cannot come into contact is not included in the cell culture region.

In one aspect, it is preferable that the coefficient of variation of the opening diameter Da of a plurality of opening pores 210 is 20% or less, and the opening ratio of the plurality of opening pores is 30% or more and 80% or less.

The major axis Db in a plane direction of the opening pore 210 is not particularly limited. For example, the average major axis of the opening pore 210 is preferably 10 $\mu$m to 750 $\mu$m, more preferably 20 $\mu$m to 300 $\mu$m, and still more preferably 30 $\mu$m to 150 $\mu$m. In a case where the average major axis of the opening pore 210 is 10 $\mu$m or more, more preferably 20 $\mu$m or more, and still more preferably 30 $\mu$m or more, a space for the presence of cells is suitably secured inside the opening pore. In a case where the average major axis of the opening pore 210 is 750 $\mu$m or less, more preferably 300 $\mu$m or less, and still more preferably 150 $\mu$m or less, it tends to be possible to spread a culture medium suitably to the cells.

The major axis Db is a major axis in a plane direction of the opening pore 210 appearing on the cut surface obtained by cutting the opening pore 210 on the major axis of the opening thereof (that is, on the opening diameter Da) and in a thickness direction. As shown in Fig. 1D, the major axis Db refers to a major axis in a plane direction in the range separated by a temporary line segment in a case where the boundary between the opening pore 210 and the communication pore 212 is separated by a temporary line segment on the cut surface. The average major axis of the opening pores 210 is an average value of the center-to-center distances for 10 or more opening pores 210 randomly selected in the observation visual field by observing the surface of the porous film 200 with a laser microscope.

The depth Dc of the opening pore 210 is not particularly limited. For example, the average depth of the opening pore 210 is preferably 10 $\mu$m to 750 $\mu$m, more preferably 20 $\mu$m to 300 $\mu$m, and still more preferably 30 $\mu$m to 150 $\mu$m. In a case where the average depth of the opening pore 210 is 10 $\mu$m or more, more preferably 20 $\mu$m or more, and still more preferably 30 $\mu$m or more, a space for the presence of cells tends to be suitably secured inside the opening pore. In addition, in a case where the average depth of the opening pore 210 is 750 $\mu$m or less, more preferably 300 $\mu$m or less, and still more preferably 150 $\mu$m or less, it tends to be possible to spread a culture medium suitably to the cells.

The depth Dc is a distance from the opening surface 206 of the opening pore 210 to the deepest portion of the opening pore 210. The average depth of the opening pore 210 is defined as an average value of the depth Dc of 10 or more opening pores 210 randomly selected in the observation visual field with a scanning electron microscope (SEM) of the cut surface in a thickness direction on the line connecting the centers of the openings.

The pore diameter Dd of the communication pore 212 is not particularly limited. For example, the average pore diameter of the communication pore 212 is preferably in a range of 50% to 500% with respect to the average major axis of hepatocytes or hepatic progenitor cells seeded on the porous film 200. In a case where the average pore diameter of the communication pore 212 is 50% or more with respect to the average major axis of the seeded hepatocytes or hepatic progenitor cells, the interaction between hepatocytes or hepatic progenitor cells present in the opening pores 210 adjacent to each other (particularly, the interaction through cell-to-cell contact) tends to be satisfactory. From the above viewpoint, the average pore diameter of the communication pore 212 is more preferably 80% or more and still more preferably 120% or more with respect to the average major axis of the seeded hepatocytes or hepatic progenitor cells. On the other hand, in a case where the average pore diameter of the communication pore 212 is 500% or less with respect to the average major axis of the seeded hepatocytes or hepatic progenitor cells, the number of cells engrafted in the communication pore 212 tends to be appropriate. From the above viewpoint, the average pore diameter of the communication pore 212 is more preferably 400% or less and still more preferably 300% or less with respect to the average major axis of the seeded hepatocytes or hepatic progenitor cells.

From the above viewpoint, the average pore diameter of the communication pore 212 is preferably in a range

of 5 μm to 50 μm, more preferably in a range of 8 μm to 40 μm, and still more preferably in a range of 12 μm to 30 μm.

The pore diameter Dd of the communication pore 212 is a major axis in a thickness direction in the contour of the communication pore 212 appearing on the cut surface in a thickness direction on the line connecting the centers of the openings. The average pore diameter of the communication pore 212 is an average value of the pore diameter Dd of 10 or more communication pores 212 randomly selected in the observation with a scanning electron microscope (SEM).

From the viewpoint of easily obtaining a homogeneous hepatocyte culture, the coefficient of variation of the pore diameter Dd of the communication pore 212 is preferably 30% or less and more preferably 20% or less, and is preferably as small as possible. The coefficient of variation is obtained for the pore diameter Dd of any 10 communication pores 212.

It is preferable that the communication pores 212 are provided at substantially the same depth (including the same depth) over an entire area in a plane direction. As a reference guideline of being substantially the same, the coefficient of variation of the distance from the opening surface 206 to the deepest portion of the communication pore 212 is preferably 10% or less on the cut surface for measuring the pore diameter Dd. The coefficient of variation is obtained for any 10 communication pores.

The width W of the flat portion 208 is preferably narrower than the major axis of the seeded hepatocytes or hepatic progenitor cells. This leads to a decreased proportion of cells cultured on the flat portion 208 and an increased proportion of cells cultured inside the opening pores 210 and the communication pores 212. The width W is a length of the flat portion 208 which is measured between the centers of the openings.

The material of the porous layer 204 is not particularly limited. The material of the porous layer 204 is preferably a hydrophobic polymer soluble in a hydrophobic organic solvent, from the viewpoint of producing the porous film 200 by a production method which will be described later. The hydrophobic organic solvent is a liquid having a solubility in water at 25°C of 10 (g/100 g water) or less.

Examples of the hydrophobic polymer include polymers such as polystyrene, polyacrylate, polymethacrylate, polyacrylamide, polymethacrylamide, polyvinyl chloride, polyvinylidene chloride, polyvinylidene fluoride, polyhexafluoropropene, polyvinyl ether, polyvinyl carbazole, polyvinyl acetate, polytetrafluoroethylene, polyester (for example, polyethylene terephthalate, polyethylene naphthalate, polyethylene succinate, polybutylene succinate, polylactic acid, or poly-3-hydroxybutyrate), polylactone (for example, polycaprolactone), polyamide or polyimide (for example, nylon or polyamide acid), polyurethane, polyurea, polybutadiene, polycarbonate, polyaromatics, polysulfone, polyethersulfone, polysiloxane derivatives, and cellulose acylate (for example, triacetyl cellulose, cellulose acetate propionate, or cellulose acetate butyrate). These polymers may be homopolymers, copolymers, polymer blends, or polymer alloys, if necessary, from the viewpoints of solubility in solvents, optical properties, electrical properties, film hardness, elasticity, and the like. These polymers may be used alone or in admixture of two or more thereof. From the viewpoint of bioabsorbability, polylactic acid, polycaprolactone, poly-3-hydroxybutyrate, and the like are preferable.

The material of the support 202 may be the same as or different from the material of the porous layer 204. Examples of the material of the support 202 include a synthetic resin, glass, and a metal. In a case where the porous layer 204 is peeled from the support 202 before or after cell culture, the material of the support 202 is preferably a material different from that of the porous layer 204.

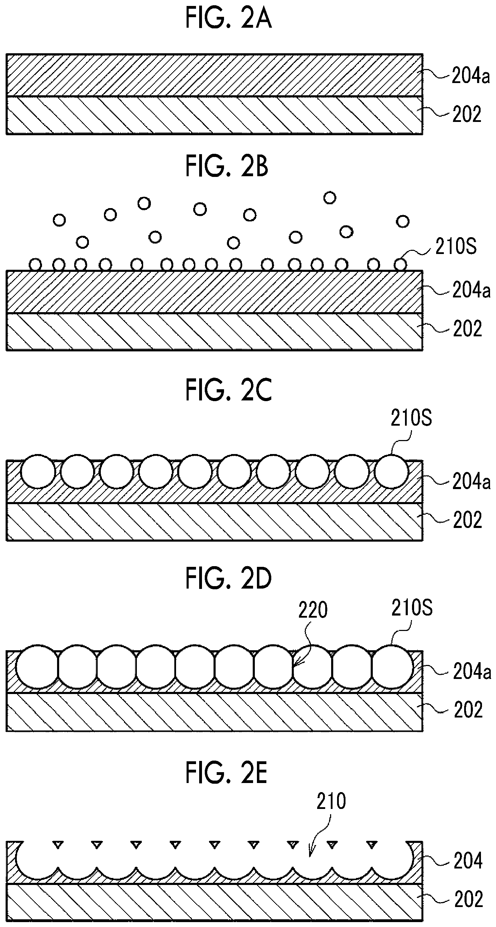

The porous film 200 is produced by, for example, a production method in which the following steps (a) to (e) are carried out in order. Fig. 2 is a schematic diagram showing a cross section of the porous film 200 in the steps (a) to (e).

[Step (a)]

A coating liquid prepared by dissolving a hydrophobic polymer constituting the porous layer 204 in a solvent is prepared and the coating liquid is applied onto the support 202 to form a coating film 204a on the support 202.

The coating liquid is prepared by mixing a hydrophobic polymer, a solvent, and an amphiphilic compound. The solvent is preferably a mixed solvent of a good solvent for a hydrophobic polymer and a hydrophobic liquid, or a hydrophobic organic solvent which is a good solvent for a hydrophobic polymer. Examples of the latter include halogen-based solvents such as trichloromethane, dichloromethane, and chloroform, in which these solvents may be used in admixture with hydrocarbon compounds such as n-hexane, cyclohexane, n-pentane, n-octane, and n-heptane which are hydrophobic liquids.

The amphiphilic compound is a compound having both a hydrophilic group and a hydrophobic group. In a case where the amphiphilic compound is formulated in a coating liquid, it is easy to form water droplets on the surface of the coating film. In addition, it is possible to more easily control the growth of water droplets by controlling the dispersion state of the hydrophobic polymer with respect to the solvent with the amphiphilic compound. Examples of the amphiphilic compound include many sorts of commercially available surfactants, oligomers such as dimers and trimers, and high molecular weight compounds such as polymers. Specific examples of the amphiphilic compound include compounds having a polyacrylic skeleton as a main chain and having a long chain aliphatic group (for example, a dodecyl group) as a lipophilic side chain and a carboxyl group as a hydrophilic side chain; polyethylene glycol/polypropylene glycol block copolymers; and phospholipids.

In the coating liquid, the concentration of the hydrophobic polymer is preferably 0.1% by mass to 10% by mass, and the concentration of the amphiphilic compound is preferably 0.01% by mass to 1% by mass.

[Step (b)]

Humidified air is allowed to flow over the entire surface of the coating film 204a to form water droplets 210S on the coating film 204a due to dew condensation. At this time, at least one of dew point Td of the humidified air or surface temperature Ts of the coating film 204a is controlled such that the difference $\Delta T$ (= Td - Ts) between the dew point Td of the humidified air flowing in the vicinity of the coating film 204a and the surface temperature Ts of the coating film 204a satisfies Expression (1).

$$\text{Expression (1): } 3°C \leq \Delta T \leq 30°C$$

[Step (c)]

Water droplets 210S are grown by continuing the supply of humidified air. Each of the water droplets 210S grows to substantially the same size (including the same size). With the evaporation of the solvent contained in the coating film 204a, the water droplets 210S are arranged in a honeycomb shape by the transverse capillary force and enter the coating film 204a. In parallel with this, as the solvent contained in the coating film 204a evaporates, the hydrophobic polymer precipitates around the water droplets 210S.

[Step (d)]

The supply of the humidified air is continued to grow the water droplets 210S until the water droplets 210S are brought into close contact with one another with amphiphilic compound films 220 interposed therebetween in the coating film 204a.

[Step (e)]

Simultaneously with or after evaporation of the solvent, the water droplets 210S are evaporated so that the portion where the water droplets 210S enter the coating film 204a is left as the opening pore 210, and in parallel, the amphiphilic compound films 220 that separate the closely adjacent water droplets 210S are destroyed. This results in the formation of the porous layer 204 in which a plurality of opening pores 210 are arranged in a honeycomb shape and connected inside.

Through the above steps (a) to (e), a laminate of the porous layer 204 and the support 202 is obtained. The laminate of the porous layer 204 and the support 202 may be used as the porous film 200; the porous layer 204 may be peeled from the support 202, and then the monolayer structure of the porous layer 204 may be used as the porous film 200; or the porous layer 204 may be peeled from the support 202 and attached to another support to form the porous film 200. A charge treatment by plasma discharge or the like may be carried out on the surface of the porous film 200 on which the cells are seeded.

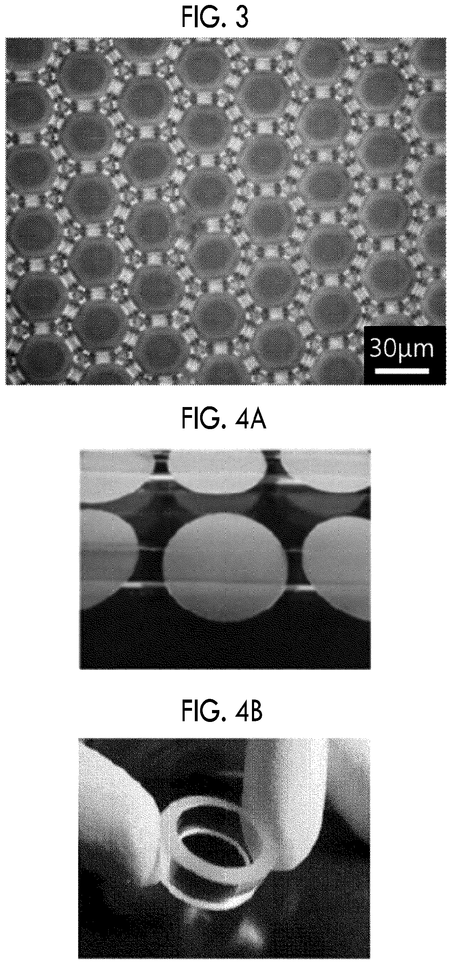

Fig. 3 shows a micrograph of an example of a porous film produced through the steps (a) to (e) taken from the side of an opening surface using a laser microscope.

The opening diameter Da, the major axis Db, and the depth Dc of the opening pore 210 and the pore diameter Dd of the communication pore 212 can be controlled by adjusting the size of the water droplets 210S. The size of the water droplets 210S can be adjusted by the film thickness of the coating film 204a, the amount of water vapor contained in the humidified air, and the timing at which the water droplets 210S are evaporated.

According to the above-mentioned production method, it is possible to produce a porous film in which a plurality of opening pores are arranged with high uniformity in a honeycomb shape without using a mold or a mask. The details of the above-mentioned production method are described in, for example, JP2007-291367A, JP2009-256624A, JP2011-74140A, and JP2011-202100A. The porous film 200 can also be produced by etching, sandblasting, press molding, or the like.

<Method for producing hepatocyte culture>

The method for producing a hepatocyte culture according to the present disclosure includes a culturing step of culturing a hepatocyte or a hepatic progenitor cell inside at least a part of opening pores and communication pores of a porous film having a plurality of opening pores provided on a surface of the porous film and communication pores for communicating the opening pores adjacent to each other. The hepatocyte culture represents a cultured hepatocyte. The details of the porous film are as described above.

According to the method for producing a hepatocyte culture according to the present disclosure, the functional characteristics of hepatocytes can be satisfactorily maintained or improved. The reason for this is not always clear, but it is presumed as follows. Since hepatocytes are cultured in the pores of a porous film in the method for producing a hepatocyte culture according to the present disclosure, it is possible to spread a culture medium to the hepatocytes, and it is possible to three-dimensionally culture the hepatocytes to secure the cell-to-cell interaction. Further, since the porous film used in the method for producing a hepatocyte culture according to the present disclosure has communication pores that communicate the opening pores adjacent to each other, it is possible for cells to interact with each other through cytokines or the like or through cell-to-cell contact even in a case where the cells are present inside the separate opening pores. Therefore, it is presumed that the method for producing a hepatocyte culture according to the present disclosure makes it possible to achieve both an advantage of two-dimensional culture and an advantage of three-dimensional culture, which contributes to the maintenance or improvement of the functional characteristics of hepatocytes.

In addition, the method for producing a hepatocyte culture according to the present disclosure also has an advantage that the function of a cell can be easily maintained or improved by culturing a hepatocyte or a hepatic progenitor cell using the porous film according to the present disclosure.

The hepatocyte or hepatic progenitor cell used for culture is not particularly limited as long as it is a hepatocyte or a cell capable of differentiating into a hepatocyte. The hepatocyte or hepatic progenitor cell may be of human origin or of non-human mammal origin. In addition, the hepatocyte or hepatic progenitor cell may be a tumor cell or a non-tumor cell.

The hepatic progenitor cell refers to a cell which has a proliferative ability and a bidirectional differentiation ability into a hepatocyte and a bile duct epithelial cell and in which E-cadherin has been identified as a surface antigen marker, and examples thereof include a hepatic stem cell and a hepatoblast. The method for obtaining the hepatic progenitor cell is not particularly limited. For example, the hepatic progenitor cell may be a primary cell collected from a living organism or may be a cell that has been induced to differentiate from a stem cell. Examples of the stem cell used for differentiation induction include a pluripotent stem cell and a mesenchymal stem cell.

The pluripotent stem cell is an undifferentiated cell having "self-renewal ability" capable of growing while maintaining an undifferentiated state and "pluripotency" capable of differentiating into all three blastodermic layers. Examples of the pluripotent stem cell include an embryonic stem cell (ES cell), an induced pluripotent stem cell (iPS cell), an embryonic germ cell (EG cell), an embryonal carcinoma cell (EC cell), a multipotent adult progenitor cell (MAP cell), an adult pluripotent stem cell (APS cell), and a multi-lineage differentiating stress enduring cell (Muse cell). In the culturing step, a differentiation-inducing factor is added to a culture medium to differentiate a pluripotent stem cell into a hepatocyte or a hepatic progenitor cell.

Above all, the hepatocyte or hepatic progenitor cell used for culturing is preferably an iPS cell-derived differentiated cell, a human primary cell, or a non-human animal-derived primary cell. Among them, an iPS cell-derived differentiated cell is more preferable from the viewpoint that the function of the cell can be satisfactorily improved. In addition, the iPS cell-derived differentiated cell is considered to be useful from the viewpoint of being able to reproduce relatively homogeneous functions and from the viewpoint of biosafety.

The culture may be single culture of either a hepatocyte or a hepatic progenitor cell, co-culture of a hepatocyte and a hepatic progenitor cell, or co-culture of a hepatocyte and a hepatic progenitor cell with a cell other than the hepatocyte and hepatic progenitor cell. In one aspect, the percentage of the cell other than the hepatocyte and hepatic progenitor cell at the time of seeding may be 20% by number or less, 10% by number or less, or 5% by number or less with respect to the total number of seeded cells.

In a case of being co-cultured with a cell other than the hepatocyte and hepatic progenitor cell, examples of the cell other than the hepatocyte and hepatic progenitor cell include a cell having a feeder ability. Examples of the cell having a feeder ability include a mesenchymal stem cell (MSC) and a fibroblast.

The culturing step is carried out, for example, by placing the porous film in a culture vessel with the opening surface facing upward, adding a cell suspension on the porous film, and culturing the cells under the culture conditions suitable for the seeded cell type. The culture vessel on which the porous film is placed may be any culture vessel known for cell culture. Examples of the material of the culture vessel include a resin (for example, polystyrene, polycarbonate, polyester, or acrylonitrile-butadiene-styrene resin), glass, ceramics, and a metal. Examples of the shape of the culture vessel include a multi-well plate, a Petri dish, a microarray plate, a tube, a flask, a roller bottle, and a bag.

The method for producing a hepatocyte culture according to the present disclosure may include, prior to the culturing step, a centrifugal treatment step of seeding the cells on the opening surface of the porous film, and then applying a centrifugal force in a direction from the surface seeded with the cells to the opposite surface to move the cells to the inside of the plurality of opening pores. Since the cells seeded on the porous film migrate into the opening pores of the porous film by the centrifugal treatment step, the proportion of the cells cultured in the pores tends to increase.

In order to facilitate carrying out the centrifugal treatment step in a case where the method for producing a hepatocyte culture according to the present disclosure includes the centrifugal treatment step, it is preferable to seed a cell suspension in which the cells are suspended in a relatively small amount of culture medium and carry out the centrifugal treatment step. In this case, the culture medium may be added to the culture vessel after the centrifugal treatment step.

In a case where the porous film is a laminate including two or more porous layers, hepatocytes or hepatic progenitor cells may be seeded in each porous layer, and then the porous layers may be stacked to prepare a laminate. Alternatively, two or more porous layers may be stacked to prepare a laminate, and then hepatocytes or hepatic progenitor cells may be seeded in the laminate.

[Culture medium and culture conditions]

The culture medium used in the method of the present disclosure is selected from known culture media used for culturing hepatocytes according to the type of cells to be cultured. Specific examples of the culture medium include culture media optimized for cell types by adding a cell growth factor to a basic medium, such as Dulbecco's Modified Eagle's Medium (DMEM), Dulbecco's Modified Eagle Medium:Nutrient Mixture F-12 (DMEM:F-12), Eagle's minimal essential medium (EMEM), Minimum Essential Medium Alpha (MEMα), and Basal Medium Eagle (BME). Such culture media are commercially available. The culture medium may be a culture medium in which a plurality of culture media are mixed. The pH of the culture medium is, for example, pH 7.0 to 8.0 and preferably pH 7.3 to 7.4.

Various ingredients commonly added, for example, a cell growth factor such as fibroblast growth factor-2 (FGF-2), transforming growth factor-β (TGF-β), epidermal growth factor (EGF), or vascular endothelial growth factor (VEGF); a vitamin or vitamin derivative such as ascorbic acid or retinoic acid; a sugar source such as glucose; an amino acid; an inorganic salt such as sodium selenite or sodium chloride; a protein such as transferrin; a hormone such as insulin; a differentiation-inhibiting factor; a differentiation-inducing factor such as dexamethasone or oncostatin M; an antioxidant such as 2-mercaptoethanol or dithiothreitol; and an antibiotic such as penicillin, streptomycin, or gentamycin, may be added to the culture medium. The ingredients may be supplemented to the culture medium during cell culture in order to keep the concentrations thereof within a desired range throughout the culture period. From the viewpoint of suppressing incorporation of an antigenic substance, an infectious source, and the like, it is preferable that the culture medium does not contain a serum and a serum substitute.

In the culturing step, it is preferable to exchange the culture medium as appropriate. The culture media used for the series of culture stages during the culturing step may not have the same composition. The culture may be continued while replacing the culture medium with a culture medium having a different composition.

The seeding density of cells relative to the porous film is, for example, $1 \times 10^3$ cells/cm$^2$ to $1 \times 10^6$ cells/cm$^2$ and preferably $1 \times 10^4$ cells/cm$^2$ to $1 \times 10^6$ cells/cm$^2$. In a case where a plurality of types of cells are co-cultured, it is preferable to set the total seeding density of the cells within the above range. The seeding density is the number of seeded cells per area of the cell culture region (that is, the total area of the flat portions and the openings) on the opening surface of the porous film.

General conditions may be applied to the culture conditions. For example, culture in an incubator at 37°C and 5% (v/v) $CO_2$ concentration is applied. The culture period is not particularly limited and may be, for example, 1 to 20 days, 3 to 18 days, or 7 to 14 days after seeding of hepatocytes or hepatic progenitor cells on a porous film.

Hepatocytes or hepatic progenitor cells may be precultured before seeding of hepatocytes or hepatic progenitor cells on the porous film. In a case where a differentiated hepatocyte is used, maturation of the cell is possible without differentiation induction, but in a case where a desired degree of maturation has not been reached, the preculture may be carried out in a differentiation induction culture medium. In a case where a hepatic progenitor cell is used, differentiation thereof into a hepatocyte is possible by carrying out the preculture in a differentiation induction culture medium.

The preculture may be carried out in a state where hepatocytes or hepatic progenitor cells are seeded on the above-mentioned porous film, or may be carried out in another culture device before seeding of hepatocytes or hepatic progenitor cells on the porous film.

The differentiation of hepatic progenitor cells can be induced by any method as long as it is possible to induce differentiation of hepatic progenitor cells into hepatocytes. For example, there is a method of culturing the cells in a culture liquid supplemented with oncostatin M, dexamethasone, a hepatocyte growth factor (HGF), and the like (Journal of Cellular Physiology, Vol. 227 (5), pp. 2051 to 2058 (2012); and Hepatology, Vol. 45 (5), pp. 1229 to 1239 (2007)).

In the present disclosure, the "maturation" of hepatocytes means that an expression level of at least one, preferably two or more, of proteins (albumin, CYP3A4, and the like) known as hepatocyte markers is improved over time. Preferably, the expression level of a protein known as a hepatocyte marker in any hepatocyte or hepatic progenitor cell cultured inside the pores of a porous film is equal to or higher than the level of a hepatocyte marker expressed in a case where a primary cell is cultured for the same period. The expression level of the hepatocyte marker can be specifically measured by the method described in Examples or a method known per se.

<Cell-containing porous film>

The cell-containing porous film according to one embodiment of the present disclosure includes a porous film having a plurality of opening pores provided on a surface of the porous film and communication pores for communicating the opening pores adjacent to each other, and hepatocytes or hepatic progenitor cells, which are present inside at least a part of the opening pores and the communication pores of the porous film. The details of the porous film and the hepatocytes or hepatic progenitor cells are as described hereinbefore.

In the cell-containing porous film of the present embodiment, the hepatocytes or hepatic progenitor cells present inside the pores of the porous film can satisfactorily maintain or improve their functional characteristics as described hereinbefore. Therefore, the cell-containing porous film of the present embodiment can be particularly useful in screening of pharmaceuticals, evaluation of efficacy, metabolic properties, or the like, functional study of hepatocytes, and the like.

In a case where hepatocytes or hepatic progenitor cells are cultured in the above-mentioned porous film, the opening pores in which the cells are present tend to be continuous in a beaded shape. Therefore, the cell-containing porous film may be one in which hepatocytes or hepatic progenitor cells are present inside two or more adjacent opening pores among the plurality of opening pores.

<Method for improving or maintaining function of hepatocyte or hepatic progenitor cell>

The method for improving or maintaining a function of a hepatocyte or a hepatic progenitor cell according to one embodiment of the present disclosure includes a culturing step of culturing a hepatocyte or a hepatic progenitor cell inside at least a part of opening pores and communication pores of a porous film having a plurality of opening pores provided on a surface of the porous film and the communication pores for communicating the opening pores adjacent to each other. The details of the culturing step are as described hereinbefore.

As described hereinbefore, culturing hepatocytes or hepatic progenitor cells inside the pores of the porous film makes it possible to satisfactorily maintain or improve the functional characteristics of the cells.

In the present disclosure, the "function" of a hepatocyte or a hepatic progenitor cell refers to a function inherent in the hepatocyte, such as protein synthesis ability and drug metabolism ability. The expression level of at least one of proteins (albumin, CYP3A4, and the like) known as hepatocyte markers can be used as an indicator.

The albumin expression level (secretion level) can be evaluated by an enzyme-linked immunosorbent assay (ELISA) using a culture medium after culturing cells for a certain period of time. Specifically, the albumin expression level can be evaluated by the method described in Examples.

The CYP3A4 activity can be evaluated, for example, by a luminescence method using a P450-Glo (registered trademark) CYP3A4 Assay (Catalog No. V9001, available from Promega Corporation). Specifically, the CYP3A4 activity can be evaluated by the method described in Examples.

In the present disclosure, improving a function of a hepatocyte or a hepatic progenitor cell means that the hepatocyte matures, that is, the expression level of at least one, preferably two or more, of proteins (albumin, CYP3A4, and the like) known as hepatocyte markers is improved.

In a case where the albumin expression level of hepatocytes or hepatic progenitor cells is improved, for example, the albumin secretion level per cellular DNA, as measured by the method described above, is preferably more than 1.0 times, more preferably 1.2 times or more, and still more preferably 1.5 times or more on Day 7, Day 10, or Day 14, based on the albumin expression level on Day 3 after seeding the cells on the porous film.

In a case where the CYP3A4 activity of hepatocytes or hepatic progenitor cells is improved, for example, the CYP3A4 activity per cellular DNA, as measured by the method described above, is preferably more than 1.0 times, more preferably 1.2 times or more, still more preferably 1.5 times or more, particularly preferably 2.0 times or more, and extremely preferably 3.0 times or more on Day 7, Day 10, or Day 14, based on the CYP3A4 activity on Day 3 after seeding the cells on the porous film.

The present inventors have found that both albumin expression level and CYP3A4 activity can be improved in a case where iPS cell-derived differentiated cells are subjected to the culturing step in pores of the above-mentioned porous film. Therefore, the method described in the present disclosure is considered to be particularly useful in a case where iPS cell-derived differentiated cells are used.

Maintaining a function of a hepatocyte or a hepatic progenitor cell means that the expression level of at least one, preferably two or more, of proteins (albumin, CYP3A4, and the like) known as hepatocyte markers of the hepatocyte or hepatic progenitor cell is maintained. Here, the "maintaining" a function means that the function is maintained without being completely lost, and does not necessarily mean that the function does not deteriorate over time.

For example, in the conventional hepatocyte primary culture system, the function of the cultured hepatocytes significantly decreased over time, and the period during which sufficient hepatocyte function was maintained was as short as several days. However, according to the method of the present disclosure, it has been found that the decrease in hepatocyte function can be suppressed even by using primary cells as compared with the conventional method.

In a case where the albumin expression level of hepatocytes or hepatic progenitor cells is maintained, for example, the albumin expression level per cellular DNA, as measured by the method described above, is preferably maintained at 0.1 times or more, more preferably 0.2 times or more, and still more preferably 0.3 times or more on Day 5, Day 7, or Day 10, based on the albumin expression level on Day 3 after seeding the cells on the porous film.

In a case where the CYP3A4 activity of hepatocytes or hepatic progenitor cells is maintained, for example, the CYP3A4 activity per cellular DNA, as measured by the method described above, is preferably maintained at 0.1 times or more, more preferably 0.2 times or more, and still more preferably 0.3 times or more on Day 5, Day 7, or Day 10, based on the CYP3A4 activity on Day 3 after seeding the cells on the porous film.

Examples

Hereinafter, embodiments of the present disclosure will be described in detail with reference to Examples. The embodiments of the present disclosure should not be interpreted restrictively by the following Examples.

The meanings of the abbreviations used in the present Examples are shown below.

- PET: polyethylene terephthalate
- PMMA: polymethyl methacrylate
- PDMS: polydimethylsiloxane
- DEX: dexamethasone
- SEM: scanning electron microscope

Hereinafter, the opening pores and the communication pores of the porous film are collectively referred to as "pores".

<< Example 1 >>

<Preparation of porous film and culture device>

[Preparation of porous film]

5 parts by mass of polylactic acid, 94.5 parts by mass of trichloromethane, and 0.5 parts by mass of a polyacrylamide-based amphiphilic polymer as an amphiphilic compound were mixed to prepare a coating liquid. The coating liquid was applied on a PET film (film thickness: 188 $\mu$m) to form a coating film on the PET film. Humidified air was allowed to flow over the entire surface of the coating film, and water droplets were formed on the coating film due to dew condensation. While continuing to supply the humidified air to grow the water droplets, the water droplets were made to enter the coating film. Polylactic acid was precipitated around the water droplets by evaporation of the solvent contained in the coating film while growing water droplets until the water droplets were closely in contact with one another with the amphiphilic compound film interposed therebetween in the coating film. Then, the supply of the humidified air was stopped, and the temperature of the atmosphere was raised to evaporate the water droplets so that the portions where the water droplets have entered the coating film were left as opening pores, whereby a porous film including a porous layer formed of polylactic acid having a plurality of opening pores arranged in a honeycomb shape and having a communicating structure and a PET film layer was obtained.

According to the film thickness of the coating film, the amount of water vapor contained in the humidified air, and the timing of evaporating the water droplets, the dimensions of the opening pores and the communication pores in the porous layer were adjusted to obtain porous films shown in Table 1.

In a case where the cross section and surface of the obtained porous film were observed using a laser microscope, the adjacent openings were spaced from each other on the opening surface of the porous film and the adjacent opening pores communicated with each other inside the porous film, in all of the porous films. In addition, all of the porous films showed that the opening pores did not penetrate through the porous film. Fig. 3 shows a laser micrograph of the surface of the porous film.

[Preparation of culture device]

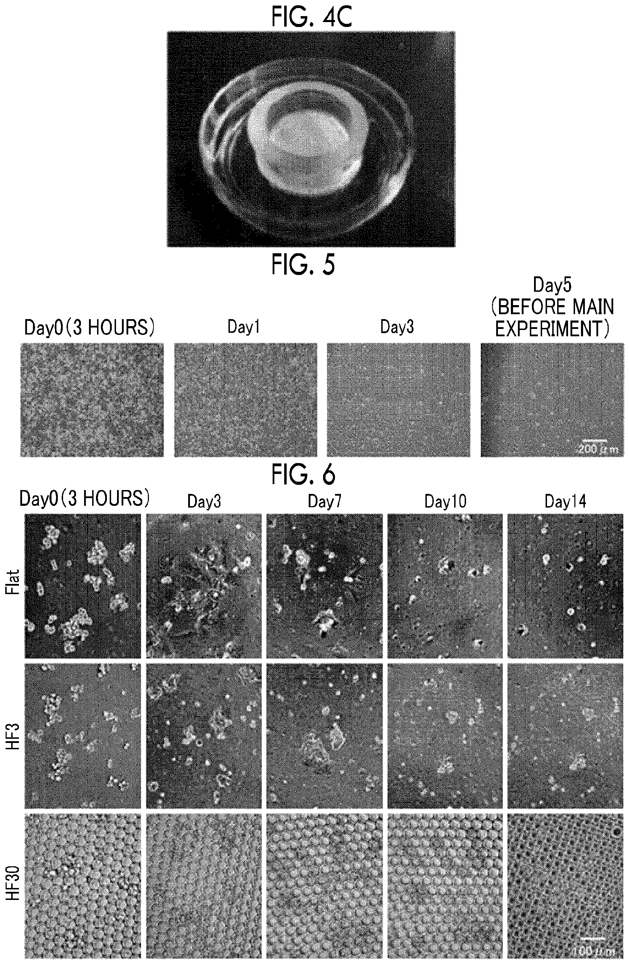

The porous film was cut into a circle having a diameter of 14 mm, and a ring (outer diameter: 14 mm, inner diameter: 9.8 mm, height: 10 mm, made of PMMA, PDMS coated on the side) was attached to the outer periphery thereof using a double-sided adhesive tape (3M product number 1510, tape for skin application) to prepare a ring-attached porous film. The ring-attached porous film was immersed in a 70% (v/v) ethanol aqueous solution for a few minutes for the purpose of sterilization and then washed with sterile water for several minutes to remove ethanol. Figs. 4A to 4C show an example of each of a porous film, a PMMA ring, and a culture device.

The ring-attached porous film was placed on a dish for tissue culture so that the PET film layer side of the porous film faced downward to obtain a culture device. For the purpose of sterilization, the culture device was irradiated with ultraviolet rays for 2 hours.

As a control culture device, a device (referred to as "Flat") was prepared in which a laminate in which a flat layer consisting of polylactic acid without opening pores was laminated on a PET film was provided instead of the porous film.

In these culture devices, the bottom surface inside the ring is the culture surface for culturing the cells, and the area of the bottom surface surrounded by the ring is 0.75 $cm^2$.

The structure of each of the prepared culture devices is shown in Table 1.

[Table 1]

| Designation of culture device | Material | Arrangement of openings | Average opening diameter | Coefficient of variation of opening diameter | Opening ratio |
|---|---|---|---|---|---|
| Flat | Polylactic acid | | - | - | - |
| HF3 | Polylactic acid | Honeycomb shape | 3.1 $\mu$m | 4.7% | 50.4% |
| HF30 | Polylactic acid | Honeycomb shape | 30.2 $\mu$m | 2.9% | 55.5% |

<Culture test>

[Preculture]

iCell Hepatocyte (hepatocyte induced to differentiate from iPS cell, single cell size: 17.4 $\pm$ 2 $\mu$m, hereinafter simply referred to as "iCell") was used as a cell to be cultured. Since iCell used for the culture was an immature cell, the iCell was matured by preculture according to the following procedure before the main culture.

iCell was seeded in a tissue culture dish coated with collagen Type I and having a diameter of 60 mm, and cultured in a differentiation induction culture medium for 5 days. Culture medium exchange was carried out daily. The detailed conditions for preculture are shown below.

- Cell seeding density: approximately 4.5 $\times$ $10^6$ cells/dish

- Differentiation induction culture medium: (DMEM:F-12) + (5 mM DEX) + (50 mg/mL gentamicin) + (10 $\mu$g/mL oncostatin M)

- Incubator: 37°C, 5% $CO_2$

Fig. 5 shows an SEM micrograph of iCell during preculture.

[Main culture]

The main culture of iCell after preculture was carried out using the three types of culture devices of Flat, HF3, and HF30 that were prepared. Culturing was carried out by the Geltrex-Mix method according to the following procedure.

In each culture device, iCell was seeded in a culture medium supplemented with 10% by volume of Geltrex (registered trademark, an extracellular matrix manufactured by Thermo Fisher Scientific Inc.) and left in the same culture medium for 2 days. Then, iCell was cultured in a culture medium to which Geltrex was not added. The culture medium exchange was carried out every 2 days, and cell culture was carried out for a total of 14 days. The detailed conditions of the main culture are shown below.

**[0123]** - Cell seeding density: 3 $\times$ $10^4$ cells/disc to 4 $\times$ $10^4$ cells/disc (4 $\times$ $10^4$ cells/$cm^2$ to 5.3 $\times$ $10^4$ cells/$cm^2$)

- Culture medium: (DMEM-F12) + (5 mM DEX) + (50 mg/mL gentamicin)

- Culture liquid volume: 0.5 mL/disc
- Incubator: 37°C, 5% $CO_2$

Using a phase contrast microscope, cellular morphological changes of iCell were observed at 3 hours and on Day 3, Day 7, Day 10, and Day 14 after the start of the main culture. Fig. 6 shows a phase contrast image of cellular morphological changes of iCell. In cases of Flat and HF3, iCell had an elongated morphology on the surface of the porous film. On the other hand, iCell present in the pores was observed in a case of HF30. In a case of HF30, the opening pores in which cells were present tended to be continuous in a beaded beaded shape.

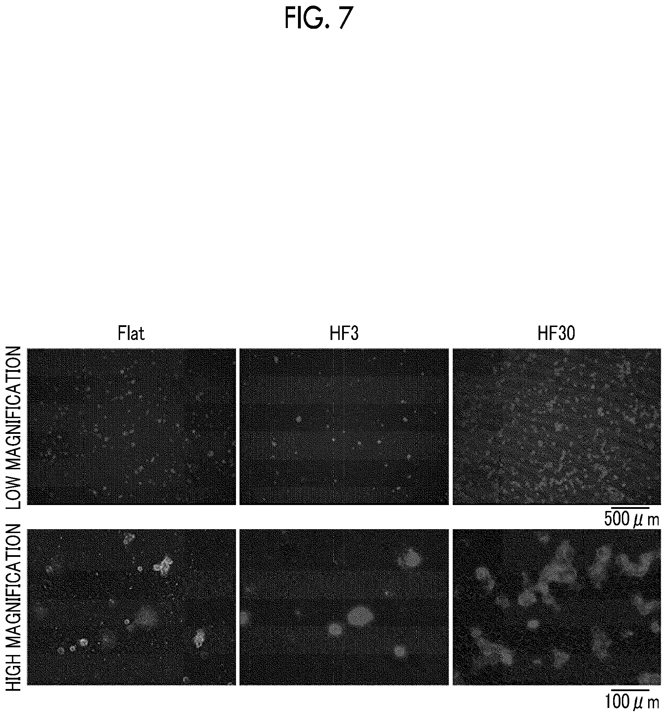

On Day 7 after the start of the main culture, the actin skeleton of iCell was stained to examine the state of cells. The results are shown in Fig. 7. It was observed that the cell retention was the highest in a case where HF30 was used.

[Measurement of amount of DNA]

The amount of DNA was measured by using a DNA-DAPI (4,6-diaminodino-2-phenylindole) fluorescence method. A calibration curve for DNA versus fluorescence intensity was prepared using salmon-derived standard DNA, and the amount of DNA extracted from cultured cells was calculated based on this relationship.

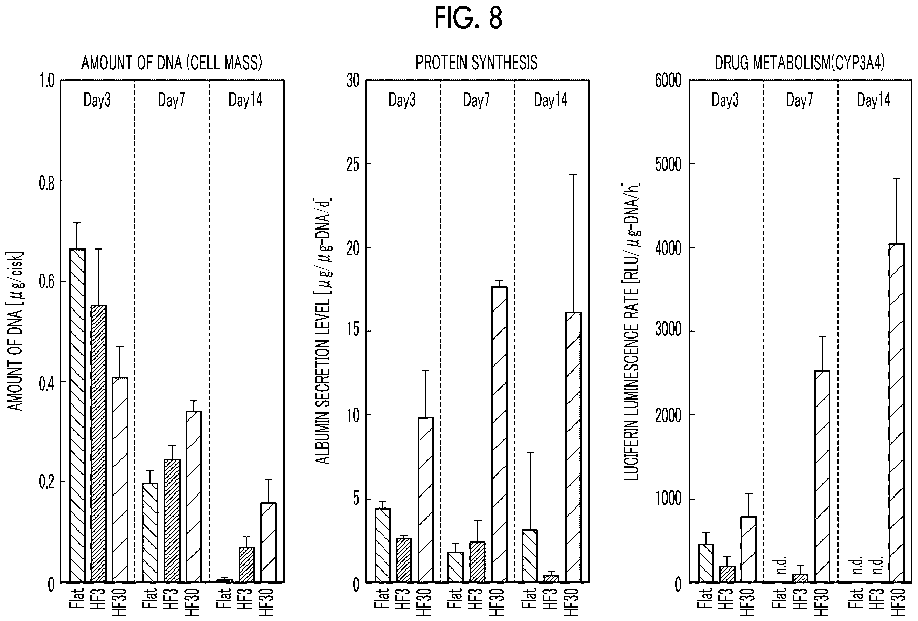

As shown in Fig. 8, in a case where HF30 was used, the cell retention of iCell on Day 7 and Day 14 after the start of the main culture was the highest. It is considered that this is because the cells are retained in the honeycomb pores in a case where HF30 is used.

[Measurement of albumin secretion level]

All the culture media cultured for a certain period of time were collected to measure the amounts of liquid. The amount of albumin secreted in the culture medium was quantified by an enzyme-linked immunosorbent assay (ELISA) and converted into a rate of albumin secretion per unit amount of DNA.

As shown in Fig. 8, in a case where HF30 was used, the albumin secretory activity was maintained at the highest level on all of Day 3, Day 7, and Day 14 after the start of the main culture.

[Measurement of CYP3A4 activity]

The CYP3A4 enzyme activity, which is a typical drug-metabolizing function of hepatocytes, was measured by a luminescence method using a P450-Glo (registered trademark) CYP3A4 Assay (Catalog No. V9001, available from Promega Corporation). Hepatocytes were incubated for 60 minutes (37°C) in a culture medium containing a luminescent substrate Luciferin-IPA. Then, a luciferin detection reagent was added and reacted for 20 minutes, the amount of luciferin produced was measured using a luminometer, and the amount of luminescence was taken as a cell activity value.

As shown in Fig. 8, in a case where HF30 was used, the activity of CYP3A4 was maintained at the highest level on all of Day 3, Day 7, and Day 14 after the start of the main culture.

From the above results, it was shown that the culture in pores of HF30 is an effective means capable of maintaining or improving the albumin secretion level and CYP3A4 activity of iCell.

<< Example 2 >>

The culture was carried out under the following conditions, using the same culture devices (Flat, HF3, and HF30) as in Example 1 as the culture devices.

- Cell to be cultured: primary rat hepatocyte (rat hepatocyte): Wister rat (7 weeks old)
- Cell seeding density: $4 \times 10^4$ cells/disk
- Culture medium: RatHep culture medium (HDM serum-free culture medium): HUVEC culture medium (EBM2 + growth factor): MSC culture medium (MSCBM) = 3:2:1 mixed culture medium (by volume ratio)
- Culture liquid volume: 0.5 mL/disk
- Incubator: 37°C, 5% $CO_2$

In order to improve the adhesiveness of cells to the porous film, only the culture medium for cell seeding was a culture medium supplemented with 10% by volume of Geltrex (culture medium:Geltrex = 9:1 (by volume ratio)). Then, centrifugation was carried out at 600 rpm for 90 seconds in order to efficiently introduce the cells into the pores of the porous film. The culture medium exchange was carried out every 2 days, and cell culture was carried out for a total of 10 days.

In a case where the morphological changes of the cells were observed with a phase contrast microscope in the same manner as in Example 1, the cells had an elongated morphology on the surface of the porous film, in cases of Flat and HF3. On the other hand, cells present in the pores of the porous film were observed in a case of HF30.

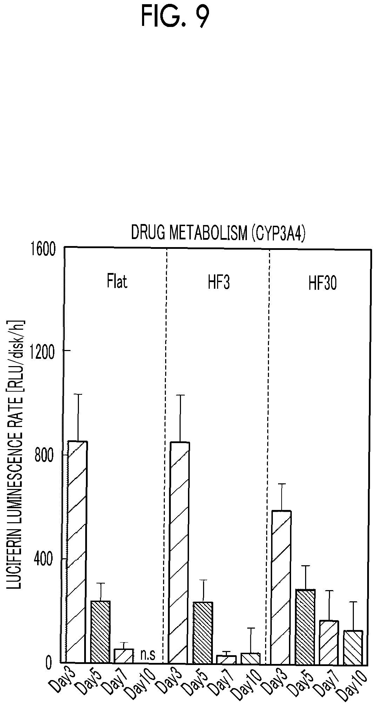

The CYP3A4 activity of the cells on Day 3, Day 5, Day 7, and Day 10 after the start of culture was measured in the same manner as in Example 1. The results are shown in Fig. 9.

As shown in Fig. 9, the CYP3A4 activity of hepatocytes was maintained even 10 days after seeding of the cells, in a case where HF30 was used as the culture device. On the other hand, in a case where Flat was used as the culture device, hepatocytes lost the CYP3A4 activity 10 days after seeding of the cells; and in a case where HF3 was used as the culture device, the CYP3A4 activity was inferior to that in a case where HF30 was used as the culture device. From the above results, it was shown that cell culture in the pores of HF30 is an effective means capable of satisfactorily maintaining the CYP3A4 activity of primary cells.

The disclosure of JP2019-120416 is incorporated herein by reference in its entirety. All documents, patent applications, and technical standards described in the present specification are incorporated herein to the same extent as the case where each individual document, patent application, and technical standard were specifically and individually indicated to be incorporated by reference.

Explanation of References

| | |
|---|---|
| 200: | porous film |
| 202: | support |
| 204: | porous layer |
| 206: | opening surface |
| 208: | flat portion |
| 210: | opening pore |
| 212: | communication pore |
| W: | width of flat portion 208 |
| PI: | center-to-center distance of opening of opening pore 210 |
| Da: | opening diameter of opening pore 210 |
| Db: | major axis in plane direction of opening pore 210 |
| Dc: | depth of opening pore 210 |
| Dd: | pore diameter of communication pore 212 |
| 204a: | coating film |
| 210S: | water droplet |
| 220: | amphiphilic compound film |

**Claims**

1. A method for producing a hepatocyte culture, the method comprising a culturing step of culturing a hepatocyte or a hepatic progenitor cell inside at least a part of a plurality of opening pores and communication pores of a porous film having the plurality of opening pores provided on a surface of the porous film and the communication pores for communicating the opening pores adjacent to each other.

2. The production method according to claim 1, wherein an average opening diameter of the plurality of opening pores is 10 $\mu$m or more and 500 $\mu$m or less.

3. The production method according to claim 1, wherein an average opening diameter of the plurality of opening pores is 20 $\mu$m or more and 200 $\mu$m or less.

4. The production method according to any one of claims 1 to 3, wherein the plurality of opening pores are arranged in a honeycomb shape on the surface of the porous film.

5. The production method according to any one of claims 1 to 4, wherein a coefficient of variation of opening diameters of the plurality of opening pores is 20% or less, and an opening ratio of the plurality of opening pores is 30% or more and 80% or less.

**6.** The production method according to any one of claims 1 to 5, wherein the porous film is a monolayer structure including one porous layer having the plurality of opening pores and the communication pores.

**7.** The production method according to any one of claims 1 to 5, wherein the porous film is a laminate including two or more porous layers having the plurality of opening pores and the communication pores, and average opening diameters of the plurality of opening pores are the same or different between the porous layers.

**8.** The production method according to any one of claims 1 to 6, wherein the porous film has a non-penetrating structure.

**9.** The production method according to any one of claims 1 to 8, wherein the hepatocyte or the hepatic progenitor cell is an iPS cell-derived differentiated cell, a human primary cell, or a non-human animal-derived primary cell.

**10.** A cell-containing porous film comprising:

a porous film having a plurality of opening pores provided on a surface of the porous film and communication pores for communicating the opening pores adjacent to each other; and
hepatocytes or hepatic progenitor cells present inside at least a part of the plurality of opening pores and the communication pores of the porous film.

**11.** The cell-containing porous film according to claim 10, wherein the hepatocytes or the hepatic progenitor cells are present inside two or more adjacent opening pores among the plurality of opening pores.

**12.** A method for improving or maintaining a function of a hepatocyte or a hepatic progenitor cell, the method comprising a culturing step of culturing a hepatocyte or a hepatic progenitor cell inside at least a part of a plurality of opening pores and communication pores of a porous film having the plurality of opening pores provided on a surface of the porous film and the communication pores for communicating the opening pores adjacent to each other.

**13.** The method for improving or maintaining a function of a hepatocyte or a hepatic progenitor cell according to claim 12, wherein an average opening diameter of the plurality of opening pores is 10 $\mu$m or more and 500 $\mu$m or less.

**14.** The method for improving or maintaining a function of a hepatocyte or a hepatic progenitor cell according to claim 12, wherein an average opening diameter of the plurality of opening pores is 20 $\mu$m or more and 200 $\mu$m or less.

**15.** The method for improving or maintaining a function of a hepatocyte or a hepatic progenitor cell according to any one of claims 12 to 14, wherein the plurality of opening pores are arranged in a honeycomb shape on the surface of the porous film.

**16.** The method for improving or maintaining a function of a hepatocyte or a hepatic progenitor cell according to any one of claims 12 to 15, wherein a coefficient of variation of opening diameters of the plurality of opening pores is 20% or less, and an opening ratio of the plurality of opening pores is 30% or more and 80% or less.

**17.** The method for improving or maintaining a function of a hepatocyte or a hepatic progenitor cell according to any one of claims 12 to 16, wherein the porous film is a monolayer structure including one porous layer having the plurality of opening pores and the communication pores.

**18.** The method for improving or maintaining a function of a hepatocyte or a hepatic progenitor cell according to any one of claims 12 to 16, wherein the porous film is a laminate including two or more porous layers having the plurality of opening pores and the communication pores, and average opening diameters of the plurality of opening pores are the same or different between the porous layers.

**19.** The method for improving or maintaining a function of a hepatocyte or a hepatic progenitor cell according to any one of claims 12 to 17, wherein the porous film has a non-penetrating structure.

**20.** The method for improving or maintaining a function of a hepatocyte or a hepatic progenitor cell according to any one of claims 12 to 19, wherein the hepatocyte or the hepatic progenitor cell is an iPS cell-derived differentiated cell, a human primary cell, or a non-human animal-derived primary cell.

# FIG. 1A

# FIG. 1B

# FIG. 1C

# FIG. 1D

EP 3 971 280 A1

## FIG. 2A

## FIG. 2B

## FIG. 2C

## FIG. 2D

## FIG. 2E

19

## FIG. 3

30μm

## FIG. 4A

## FIG. 4B

## FIG. 4C

## FIG. 5

| Day0(3 HOURS) | Day1 | Day3 | Day5 (BEFORE MAIN EXPERIMENT) |

## FIG. 6

| | Day0(3 HOURS) | Day3 | Day7 | Day10 | Day14 |

Flat

HF3

HF30

# FIG. 7

FIG. 8

AMOUNT OF DNA (CELL MASS)

PROTEIN SYNTHESIS

DRUG METABOLISM(CYP3A4)

EP 3 971 280 A1

# FIG. 9

DRUG METABOLISM (CYP3A4)

Flat    HF3    HF30

LUCIFERIN LUMINESCENCE RATE [RLU/disk/h]

Day3 Day5 Day7 Day10 Day3 Day5 Day7 Day10 Day3 Day5 Day7 Day10

n.s

## INTERNATIONAL SEARCH REPORT

| | International application No. |
|---|---|
| | PCT/JP2020/023362 |

**A. CLASSIFICATION OF SUBJECT MATTER**
Int. Cl. C12M1/00(2006.01)i, C12M3/00(2006.01)i, C12N5/071(2010.01)i,
C12N5/10(2006.01)n
FI: C12N5/071, C12M1/00 A, C12M3/00 A, C12N5/10
According to International Patent Classification (IPC) or to both national classification and IPC

**B. FIELDS SEARCHED**

Minimum documentation searched (classification system followed by classification symbols)
Int. Cl. C12M1/00, C12M3/00, C12N5/071, C12N5/10

Documentation searched other than minimum documentation to the extent that such documents are included in the fields searched
Published examined utility model applications of Japan      1922-1996
Published unexamined utility model applications of Japan    1971-2020
Registered utility model specifications of Japan            1996-2020
Published registered utility model applications of Japan    1994-2020

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2018/061846 A1 (FUJIFILM CORP.) 05 April 2018, claims, paragraphs [0022], [0030], [0031], [0063], [0064], [0085], [0113]-[0123], table 1 | 1-20 |
| X | JP 2008-199962 A (FUJIFILM CORP.) 04 September 2008, claims, paragraph [0020], examples 1-3, fig. 1-14 | 1-20 |

☐ Further documents are listed in the continuation of Box C.   ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 30.07.2020 | 11.08.2020 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**
Information on patent family members

International application No.

PCT/JP2020/023362

| Patent Documents referred to in the Report | Publication Date | Patent Family | Publication Date |
|---|---|---|---|
| WO 2018/061846 A1 | 05.04.2018 | US 2019/0201585 A1 claims, paragraphs [0071], [0079], [0080], [0119], [0120], [0143], [0223]-[0251], table 1 EP 3521417 A1 | |
| JP 2008-199962 A | 04.09.2008 | US 2008/0215073 A1 claims, paragraph [0048], examples 1-3, fig. 1-14 EP 1961809 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007319006 A
- JP 2016063948 A
- WO 2018061846 A
- JP 2007291367 A
- JP 2009256624 A
- JP 2011074140 A
- JP 2011202100 A
- JP 2019120416 A

**Non-patent literature cited in the description**

- *Journal of Cellular Physiology,* 2012, vol. 227 (5), 2051-2058
- *Hepatology,* 2007, vol. 45 (5), 1229-1239